# EUROPEAN PATENT SPECIFICATION

(11) **EP 3 395 487 B1**
(45) Date of publication and mention of the grant of the patent: **22.12.2021**
(21) Application number: 18168838.3
(22) Date of filing: 23.04.2018
(51) Int. Cl.: B23K 9/095, A61F 9/06, B23K 9/10, B23K 9/32

(54) **WELDING HELMET WITH TEMPERATURE DETECTOR, AND WELDING SYSTEM WITH SUCH AND WELDING HELMET**
SCHWEISSHELM MIT TEMPERATURDETEKTOR, UND SCHWEISSSYSTEM MIT EINEM SOLCHEN HELM
CASQUE DE SOUDAGE AVEC UN DÉTECTEUR DE TEMPÉRATURE, ET SYSTEME DE SOUDAGE AVEC UN TEL CASQUE

(30) Priority: 21.04.2017 US 201715493569
(43) Date of publication of application: 31.10.2018
(73) Proprietor: Lincoln Global, Inc., Santa Fe Springs, CA 90670 (US)
(72) Inventor: NARAYANAN, Badri K., Highland Heights, OH Ohio 44143 (US); CHANTRY, Bruce John, Solon, OH Ohio 44139 (US); MUZILLA, David John, Wadsworth, OH Ohio 44281 (US)
(74) Representative: Grosse Schumacher Knauer von Hirschhausen

(56) References cited:
- KR-B1- 101 219 182
- US-A1- 2010 288 734
- US-A1- 2013 291 271
- US-A1- 2014 210 987

## Description

Embodiments of the present invention relate to a welding helmet and to a welding system according to the preamble of claim 1 and the claim 6 respectively. and thus generally to controlling a heat input for a weld or metal deposition process by monitoring thermal characteristics of a weld environment.

### Background of the Invention

Heat input into a weld is an important consideration. Often a maximum heat input level is determined and then the appropriate welding waveform and parameters are selected for the weld. However, it is difficult to change these parameters or the heat input during welding. US 2013/291271 A1 (basis for the preamble of claim 1) discloses a helmet with a temperature sensor for auto-darkening of the helmet lens. US 2010/288734 A1 discloses a welding machine with a thermal analysis. KR 101 219 182 B1 discloses a general protective helmet. Accordingly, an improved welding methodology and/or system addressing these concerns is needed.

### Summary of the Invention

The invention provides a welding helmet and a welding system according to claim 1 respectively 6. In accordance with an embodiment of the present invention, a welding system is provided that includes a power supply configured to output a welding current to an electrode to create an arc between the electrode and a workpiece. The system can further include a welding torch for performing a welding operation on the workpiece to create a weld joint, the welding torch includes the electrode. The system further includes a temperature sensor configured to detect a temperature of the workpiece along a path relative to the weld joint or a travel path during the welding operation. The system can further include a controller configured to communicate an indication to a feedback device when the temperature of the workpiece exceeds a tolerance associated with a material of the workpiece.

In accordance with an embodiment of the present invention, a welding helmet is provided that includes a shell having a filtered viewing window. The shell is configured to be worn by a human welder to protect the human welder as the human welder views a weld environment through the viewing window during a welding operation performed by the human welder using a welding system. The welding helmet includes a thermal sensing device integrated with the shell and configured to sense thermal energy of the weld environment and generate thermal data based on the thermal energy during the welding operation. The weld environment includes at least one of a workpiece, an electrode, and a weld puddle. The welding helmet includes a thermal analysis module integrated with the shell and operatively connected to the thermal sensing device. The thermal analysis module is configured to analyze the thermal data to generate control parameters. The welding also includes a transmitter device integrated with the shell and operatively connected to the thermal analysis module. The transmitter device is configured to transmit the control parameters to the welding system. The control parameters control at least one welding parameter of the welding system during the welding operation. The thermal data includes according to the present invention, can include, spatial thermal gradients and temporal thermal gradients of the weld environment. The control parameters can include, for example, a current adjustment command, a voltage adjustment command, a waveform adjustment command, or a wire feed speed adjustment command. The welding parameters can include, for example, the welding current, a welding voltage, or a wire feed speed. In one embodiment, the welding system includes a rechargeable battery integrated with the shell that is configured to provide electrical energy to the thermal sensing device, the thermal analysis module, and the transmitter device. In one embodiment, the thermal analysis module is configured as a look-up-table (LUT) stored in a memory, using the thermal data (or data derived from the thermal data) as an addressable input to the memory and providing the control parameters as an output of the memory. In one embodiment, the thermal analysis module includes a processor, a memory, and an algorithm implemented as a set of computer-executable instructions stored in the memory and configured to execute on the processor. In one embodiment, the thermal analysis module includes an electronic circuit configured to use the thermal data (or data derived from the thermal data) as an input to the electronic circuit and provide the control parameters as an output of the electronic circuit. In other embodiments the thermal analysis module may include a combination of two or more of a LUT, an electronic circuit, and an algorithm, for example. In one embodiment, the welding helmet includes an antenna operatively connected to the transmitter device. The transmitter device is configured to wirelessly transmit the control parameters to a controller or a power source of the welding system via the antenna. In an alternative embodiment, the welding helmet includes a control cable configured to be connected between the transmitter device and a controller or a power source of the welding system. The transmitter device is configured to transmit the control parameters to the controller or the power source of the welding system via the communication cable.

These and other objects and embodiments of this invention will be evident when viewed in light of the drawings, detailed description and appended claims.

### Brief Description of the Drawings

The invention may take physical form in certain parts and arrangements of parts, a preferred embodiment of which will be described in detail in the specification and illustrated in the accompanying drawings which form a part hereof, and wherein:
Fig. 1 illustrates an exemplary, non-limiting embodiment of a welding system that reduces inconsistencies in heat input based on a measured temperature of a workpiece at a distance adjacent to a travel path of the welding torch;
Fig. 2 illustrates an exemplary, non-limiting embodiment of a welding system that can detect a temperature at a location on the workpiece to reduce heat input inconsistencies during a welding operation;
Fig. 3 is a top view of the welding system illustrated in Fig. 2;
Fig. 4 illustrates an exemplary, non-limiting embodiment of a welding torch that includes a temperature sensor to detect temperature at a location that is a distance from an arc created between an electrode and a workpiece;
Fig. 5 illustrates an exemplary, non-limiting embodiment of a welding system that communicates an indication to a feedback device based on a measured temperature of a workpiece at a distance adjacent to a travel path of the welding torch;
Fig. 6 illustrates an exemplary, non-limiting embodiment of a welding controller according to one or more aspects;
Fig. 7 is a flow diagram of detecting temperature of a workpiece to reduce inconsistency of heat input for a welding operation;
Fig. 8 is a flow diagram of communicating feedback based on a monitored temperature of a workpiece at a location that is a distance from the arc created on such workpiece;
Fig. 9 illustrates a workpiece in accordance with the subject innovation;
Fig. 10 illustrates a graph in accordance with the subject innovation;
Fig. 11 illustrates a graph in accordance with the subject innovation;
Fig. 12 illustrates a predicted heat contour in accordance with the subject innovation;
Fig. 13 illustrates a graph in accordance with the subject innovation;
Fig. 14 illustrates a graph and chart in accordance with the subject innovation;
FIG. 15 illustrates a welding system for controlling welding parameters based on thermal characteristics of a weld environment;
FIG. 16 illustrates a welding helmet that is a part of the welding system of FIG. 15;
FIG. 17 illustrates an embodiment, according to the present invention, of a welding system for controlling welding parameters based on thermal characteristics of a weld environment;
FIG. 18 illustrates possible cases of a thermal analysis module that is a part of the welding system of FIG. 15 or the welding system of FIG. 17; and
FIG. 19 illustrates a graphical representation of an example of thermal data generated by a thermal sensing device of the welding system of FIG. 15 or the welding system of FIG. 17.

### Detailed Description of the Invention

Some embodiments of the present invention relate to methods and systems that relate to semi-automatic or manual welding and detecting a base material (e.g., workpiece) temperature, communicating the base material temperature, and adjusting the welding operation based on the detected base material temperature. A temperature sensor can detect temperature at a location that is a distance from the arc and, for instance, parallel to a direction of travel of the welding torch and such detected temperature can be communicated to the user performing the weld or the user controlling the semi-automatic welding system. The user can be informed if the detected temperature is within a tolerance or approved range or if the detected temperature is outside the tolerance or approved range for the type of base material and/or pre-defined temperature. Each base material or workpiece can have a corresponding temperature and distance from the center of the arc created that should be maintained for a quality weld. For instance, upon notification or indication of the detected temperature being outside a tolerance, the user can adjust a travel speed or a wire feed speed to compensate for the temperature of the base material cooling too quickly or too slowly. The detected temperature or the indication can be communicated to the user via a feedback device that provides communication such as, but not limited to, auditory, visual, adaptive, haptic, among others. The feedback device can be, but is not limited to, a helmet, gloves, an electronic device, a wearable electronic device, or an apron. In a particular embodiment of the subject innovation, the indication can be a green light for within tolerance and a red light for outside tolerance, and such light can be flashed in a peripheral vision of the user via the helmet or welding lens of a helmet.

"Welding" or "weld" as used herein including any other formatives of these words will refer to depositing of molten material through the operation of an electric arc including but not limited to submerged arc, GTAW, GMAW, MAG, MIG, TIG welding, any high energy heat source (e.g., a laser, an electron beam, among others), or any electric arc used with a welding system. Moreover, the welding operation can be on a workpiece that includes a coating such as, but not limited to, a galvanized coating.

"Component" as used herein can be a portion of hardware, a portion of software, or a combination thereof that can include or utilize at least a processor and a portion of memory, wherein the memory includes an instruction to execute.

"Tolerance" as used herein including any other formatives of the word will refer to an allowable amount of variation of a specified quantity for a value. In particular, a tolerance can be defined (e.g., dynamically, predefined, among others) for temperature for a given location for a type of workpiece for a type of welding operation. It is to be appreciated that the tolerance can be chosen with sound engineering judgment and/or by one of ordinary skill in the art without departing from the scope of the subject innovation.

While the embodiments discussed herein have been related to the systems and methods discussed above, these embodiments are intended to be exemplary and are not intended to limit the applicability of these embodiments to only those discussions set forth herein. The control systems and methodologies discussed herein are equally applicable to, and can be utilized in, systems and methods related to arc welding, laser welding, brazing, soldering, plasma cutting, waterjet cutting, laser cutting, and any other systems or methods using similar control methodology, without departing from the spirit or scope of the above discussed inventions. The embodiments and discussions herein can be readily incorporated into any of these systems and methodologies by those of skill in the art. By way of example and not limitation, a power supply as used herein (e.g., welding power supply, among others) can be a power supply for a device that performs welding, arc welding, laser welding, brazing, soldering, plasma cutting, waterjet cutting, laser cutting, among others. Thus, one of sound engineering and judgment can choose power supplies other than a welding power supply departing from the intended scope of coverage of the embodiments of the subject invention.

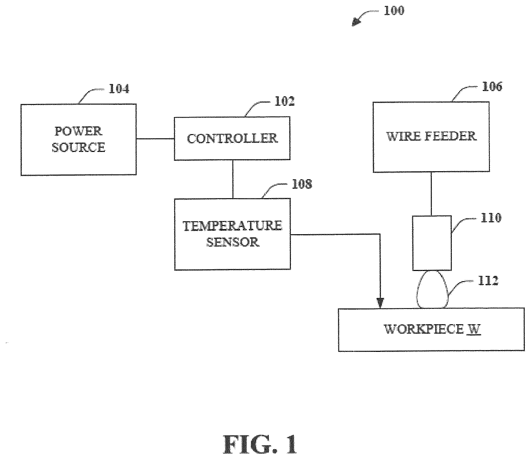

The best mode for carrying out the invention will now be described for the purposes of illustrating the best mode known to the applicant at the time of the filing of this patent application. The examples and figures are illustrative only and not meant to limit the invention, which is measured by the scope and spirit of the claims. Referring now to the drawings, wherein the showings are for the purpose of illustrating an exemplary embodiment of the invention only and not for the purpose of limiting same, Figs. 1-5 illustrates a welding system that is used with an automated or semi-automated welding system. Turning to Fig. 1, an exemplary, non-limiting embodiment of welding system 100 is illustrated that reduces inconsistencies in heat input based on a measured temperature of a workpiece at a distance adjacent to a travel path of the welding torch. System 100 includes welding torch 110 (also referred to as "torch") having an electrode in which power source 104 creates arc 112 between electrode and workpiece W to complete an electrical circuit to perform the welding operation. System 100 can include power source 104 that is configured to create arc 112 between an electrode and workpiece W and further include wire feeder 106 is configured to deliver welding wire to a puddle formed by the electrode. Controller 102 can be configured to manage wire feed speed (WFS) of wire feeder 106, power source 104 that creates arc 112 for the welding operation. It is to be appreciated that the system 100 can be used to perform a welding operation with a consumable electrode, a non-consumable electrode, a shielding gas, no shielding gas, or a combination thereof.

Heat input for a welding operation can be a critical factor for a weld created but also a workpiece W after a weld has been created on such workpiece W. For instance, some applications or industries require consistent heat input along a length of a weld in order to avoid inconsistencies in a weld created, on the workpiece W, and/or in a composition of the workpiece W. If heat input is not consistent, a defect can emerge by cooling too fast causing internal cracking for workpiece W, internal stress for workpiece W, and/or harden workpiece W. Further, defects may emerge for inconsistent heat input by cooling too slow causing increased pores in workpiece W. System 100 allows for increasing consistency in a weld as well as workpiece W after a weld has been created.

System 100 further includes temperature sensor 108 that is configured to detect a temperature at a location or a path that is adjacent to a travel path of the welding operation. By detecting or measuring a temperature at a location or a path adjacent to a travel path of the welding operation, system 100 can determine heat input for a weld created as well as for workpiece W. In particular, measuring a temperature at a location on the arc or at the arc is problematic due to the intense heat input as well as variables surrounding such location. Rather, system 100 measures temperature at a location that is a distance away from the arc in which such distance has a tolerance for a temperature dependent upon at least one of a type of material for workpiece W, a location of where the temperature is being measured, a type of welding operation being performed, a welding parameter, among others. Thus, by calculating tolerances for a temperature for a location on workpiece W having a type of material, temperature sensor 108 detects temperature at a location in which controller 102 generates an indication if the temperature detected exceeds a tolerance.

Temperature sensor 108 can measure a temperature of a workpiece W at a location on workpiece W. By way of example and not limitation, the location on workpiece W can be adjacent to at least one of arc 112, welding torch 110, electrode, a weld created, a travel path of electrode, a travel path of torch head 110, a pre-defined path for an anticipated weld to be created, or a combination thereof. As discussed in more detail below, temperature sensor 108 can be configured to identify a temperature of workpiece W during a welding operation. In an embodiment, temperature sensor 108 can be configured to identify a temperature of workpiece W at a time, wherein the time can be, but is not limited to being, before a welding operation, during a welding operation, after a welding operation, or a combination thereof.

It is to be appreciated that controller 102 can be configured to communicate an indication when a tolerance is exceeded for a temperature at a location, wherein the tolerance can be based on at least one of a time a temperature is obtained, a type of material of workpiece W, a type of welding operation, a type of electrode (e.g., consumable, non-consumable, composition of electrode, among others), a welding parameter, a distance from a location, a location on the workpiece, among others, or a combination thereof.

Temperature sensor 108 is configured to measure, detect, or identify a temperature of workpiece W. Temperature sensor 108 can be, but is not limited to being, an infrared temperature device, a thermography device, a thermal camera, a thermocouple, a thermistor, a resistance temperature detector (RTD), a remote sensor, a wireless sensor, a wireless device, a transmission and reception system with a temperature sensor, a pyrometer, a Langmuir probe, a thermometer, one or more devices that communicate temperature information or data for a location, among others. Moreover, although temperature sensor 108 is depicted as a stand-alone sensor, temperature sensor 108 or a portion of the temperature sensor 108 can be incorporated into at least one of controller 102, torch head 110, wire feeder 106, power source 104, workpiece W, electrode, or a combination thereof. For example, temperature sensor 108 can include a temperature sensor system in which the temperature sensor obtains temperature data, the temperature data is communicated to a component, wherein the component can be controller 102 or a component that communicates the temperature data to controller 102. It is to be appreciated that such communication of temperature data from temperature sensor 108 can be wired, wireless, or via one or more components (e.g., transmitter, receiver, among others).

Temperature sensor 108 can detect an actual temperature of workpiece W, wherein such detection can be at or on a location of the workpiece W. It is to be appreciated that system 100 can include one or more temperature sensors 108 which measure temperatures at one or more locations of workpiece W.

Controller 102 can be configured to communicate an indication when a temperature measured, detected, or obtained, exceeds a tolerance for the welding operation. As discussed above, the tolerance can be determined based on at least one of a time a temperature is obtained, a type of material of workpiece W, a type of welding operation, a type of electrode (e.g., consumable, non-consumable, composition of electrode, among others), a welding parameter, a distance from a location, a location on the workpiece, among others, or a combination thereof. The indication can be, but is not limited to being, audible, haptic feedback, visual, electronic communication (e.g., text message, electronic mail, displayed text, telephone communication, cellular communication, among others), or a combination thereof.

Additionally or alternatively, controller 102 can adjust a welding parameter based on the detected temperature exceeding a threshold. In a particular non-limiting example, the indication can be to an operator performing the welding operation to adjust a travel speed of the welding torch 110. For instance, for a tolerance that is exceeded due to a detected temperature above a maximum temperature for the welding operation, the indication can be to increase travel speed. In another instance, for a tolerance that is exceeded due to a detected temperature below a minimum temperature for the welding operation, the indication can be to decrease travel speed. In still another non-limiting example, controller 102 or operator performing the welding operation can adjust a wire feed speed via wire feeder 106, wherein the adjustment is based on the communicated indication. For instance, for a tolerance that is exceeded due to a detected temperature above a maximum temperature for the welding operation, the indication can be to decrease wire feed speed. In another instance, for a tolerance that is exceeded due to a detected temperature below a minimum temperature for the welding operation, the indication can be to increase wire feed speed.

By way of example, the welding parameter can be, a type of welding operation, a type of shielding gas, a material composition of workpiece W, a welding pattern, a type of electrode, a composition of electrode, a wire feed speed, a waveform used for the welding operation, a polarity of a welding wire, a type of flux, a number of electrodes used in the welding operation, an arc voltage, a travel speed of a tractor welder that performs the welding operation, a travel speed of a torch that performs the welding operation, an arc current level, a height of torch, a distance between workpiece W and torch or an end of the electrode, an oscillation width of electrode, a temperature of welding wire, a temperature of electrode, a type of material of workpiece W, a frequency of oscillation of electrode, a polarity of the arc current, a polarity of the current for welding wire, a parameter that affects an arc current of the welding operation, a gauge of wire, a material of wire, an oscillation dwell, a left oscillation dwell, a right oscillation dwell, one or more temperatures of workpiece W at one or more locations on workpiece W, a temperature of workpiece W, any and all variation of advanced process controls (e.g., move controls, pulse-frequency, ramp rates, background level ratios, etc.), and the like.

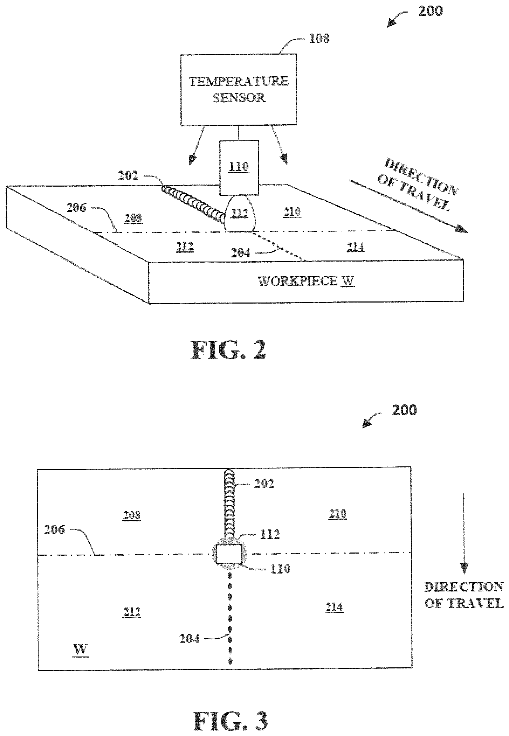

Fig. 2 illustrates an exemplary, non-limiting embodiment of welding system 200 that detects a temperature at a location on the workpiece to reduce heat input inconsistencies during a welding operation. Fig. 3 illustrates a top view of Fig. 2, yet Fig. 3 does not illustrate temperature sensor 108 so as to not obstruct the top view. Welding system 200 illustrates a portion of system 100 for the sake of brevity, yet it is to be appreciated controller 102, power source 104, and/or wire feeder 106 are utilized to create arc 112 between an electrode and workpiece W. Welding system 200 creates arc 112 to create weld 202 on workpiece W in which a travel direction indicates travel path 204, wherein travel path 204 is where a weld is to be created. Travel path 204 can be a reference to identify a first side and a second side of the workpiece in which the first side is area that includes at least location 208 and location 212 and the second side (opposite the first side) is area that includes location 210 and location 214.

Temperature sensor 108 can be configured to detect temperature at a location on workpiece W, wherein controller 102 (shown in at least Fig. 1) communicates an indication if such detected temperature exceeds a tolerance. By way of example and not limitation, temperature sensor 108 is affixed or coupled to torch head 110. Yet, it is to be appreciated that, temperature sensor 108 can be affixed or coupled to at least one of a workpiece W, a support structure or device coupled to workpiece W or a portion of a welding system, a welding equipment, among others.

Temperature sensor 108 can detect temperature at a location on workpiece W, and in particular on a surface of workpiece W in a location that is on at least one of the first side, the second side, or a combination thereof. For instance, the temperature can be detected at a distance away or from at least one of arc 112, torch head 110, weld 202, travel path 204, or a combination thereof. By way of example, and not limitation, the temperature can be detected by temperature sensor 108 at one or more of a point in location 208, a point in location 212, a point in location 210, a point in location 214, a point along travel point 204, a point along weld 202, a point along reference numeral 206, a point below a surface weld 202 is created, an underside of workpiece W, an edge of workpiece W, among others.

By way of example, the temperature can be detected at a location that is aligned with at least one of arc 112, torch head 110, or electrode as indicated by reference numeral 206. In another example, the temperature can be detected at a location that is a distance from at least one of arc 112, torch head 110, or electrode and aligned with at least one of the arc 112, torch head 110, or electrode as indicated by reference numeral 206. This allows temperature sensor 108 to detect temperature at a location that is a distance from arc 112 and aligned with reference numeral 206 which is along a path that is parallel to travel path 204 and/or direction of travel for welding system 200.

In another example, the temperature can be detected at location 208 (on the first side) or location 210 (on the second side) that is a distance from at least one of arc 112, torch head 110, or electrode and behind (e.g., lagging) the reference numeral 206. In another example, the temperature can be detected at location 212 (on the first side) or location 214 (on the second side) that is a distance from at least one of arc 112, torch head 110, or electrode and in front (e.g., leading) the reference numeral 206.

In still another example, the temperature can be detected at a location that is located on at least one of weld 202 or travel path 204. In such example, controller 102 can communicate an indication when the temperature exceeds a tolerance for such welding operation and/or type of material of workpiece W.

In another embodiment, system 200 can utilize two or more tolerances corresponding to two or more distances for a welding operation to glean heat input for a welding operation and to adjust the welding operation via controller 102 or a notification or indication to an operator in real time. Temperature sensor 108 can be configured to detect a first temperature a first distance from arc 112 in line with or along either weld 202 or travel path and a second temperature a second distance from arc 112 in line with or along reference numeral 206. Controller 102 can communicate an indication when 1) the detected first temperature exceeds a first tolerance for the first distance of the welding operation; and 2) the detected second temperature exceeds a second tolerance for the second distance of the welding operation. Such indication can be to an operator to adjust a travel speed for welding torch 110 or a wire feed speed. In another embodiment, controller 102 can adjust the travel speed or the wire feed speed based on the one or more exceeded tolerances for the detected temperatures.

In another embodiment, temperature sensor 108 can be configured to detect temperature a depth below the surface of workpiece W. It is to be appreciated that system 200 can detect temperature on any surface of workpiece W or inside workpiece W. Moreover, a tolerance for each depth or location in or on workpiece W can be used for temperature to determine whether controller 102 communicates an indication.

Temperature sensor 108 can detect one or more temperatures at one or more locations, wherein each of the one or more locations can have a respective temperature tolerance which defines at least one of a maximum temperature, a minimum temperature, a range of temperatures, among others. The respective temperature tolerance can be specific to a distance or a location on or within workpiece W. In still another example, the temperature tolerance can be particular to a type of welding operation and/or a type of material of workpiece W. By way of example and not limitation, below is a table that illustrates tolerances in accordance with the subject innovation. It is to be appreciated that the below table is not exclusive and is solely an example since tolerances in accordance with the subject innovation can be determined for various parameters as discussed above.

In another embodiment, system 200 can utilize multiple temperature sensors (e.g., more than one temperature sensor 108). In still another embodiment, system 200 can include multiple metal deposition sources that deposit material onto a workpiece. In such example of having multiple metal depositions sources, such metal depositions sources can work in series. For example, a system can include two (2) metal deposition sources, wherein a first metal deposition source can deposit material first, and upon completion or during the same time, a second metal deposition source can deposit material after.

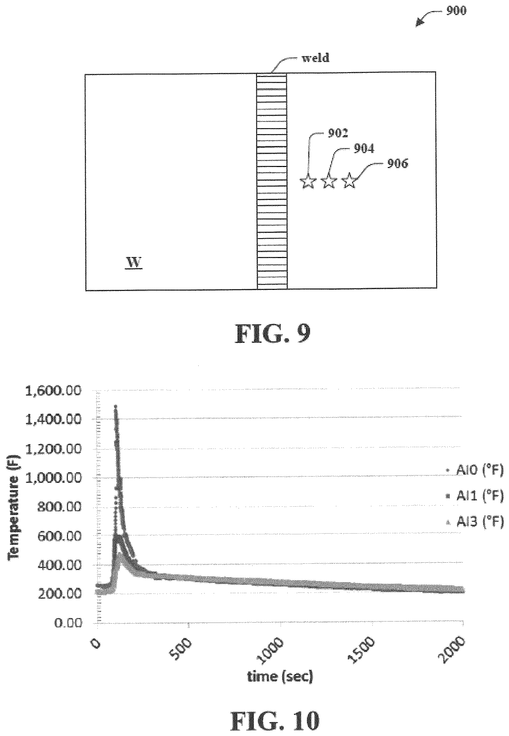

Turning to Figs. 9-14, various embodiments of the subject innovation are discussed. Fig. 9 illustrates a top view of workpiece W having metal deposited (e.g., a weld) in which workpiece W has first temperature sensor (AI1) 902, second temperature sensor (AI2) 904, and third temperature sensor (Al3) 906. Fig. 10 illustrates a graph of temperature and time for each temperature sensor. Below is Table 1 which illustrates more details for each:

**TABLE 1**

| Distance from Weld (mm) | 13. | 23. | 33. |
|---|---|---|---|
| Distance from Weld (in) | 0.53 | 0.94 | 1.3 |
| Thermo Couple | Al1 (F) | Al2 (F) | Al3 (F) |
| Max Temp (F) | 1482 | 594 | 459 |
| Max Temp (C) | 805. | 312. | 237. |

The metal deposition process for the above can be a 1 inch wide bead (e.g., add .5 in total distance from weld. In addition, the location of the temperature sensors (e.g., thermocouples) can be approximately six (6) inches to nine (9) inches from end plate dimensions ¾ inches thick, 16 inches long, and 17 inches wide. In particular, the location can be 7.5 inches from the end plate.

Below is Table 2 which shows information related to the predictive assessments. It is noted that the modeling of weld pools based on welding process parameters may have deviations from actual measurements.

**TABLE 2**

| | | | | | |
|---|---|---|---|---|---|
| Voltage | 22 | 15.7 | 19.4 | 16.1 | 18.9 |
| Current | 117 | 146 | 212 | 107 | 113 |
| Power | 3507 | 2782 | 5415 | 2391 | 3017 |
| Approx. Heat Inpu (kj/in) | 4.2 | 4.8 | 6.5 | 2.9 | 3.6 |
| Bead Width (in) | 0.21 | 0.27 | 0.28 | 0.16 | 0.19 |
| Bead width (mm) | 5.3 | 6.8 | 7.1 | 4.1 | 4.7 |
| Bead width (mm) - predicted | 3.9 | 4.3 | 4.7 | 2.9 | 3.5 |

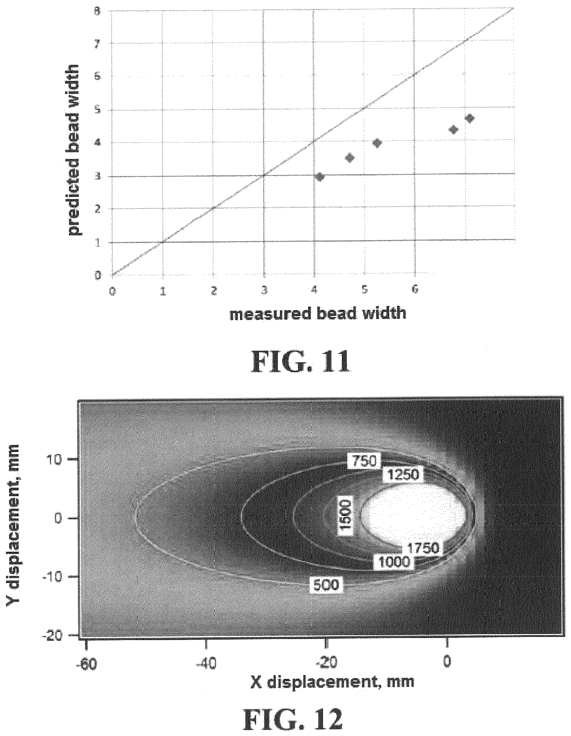

Fig. 11 illustrates a predicted bead width graph versus measured bead width. Fig. 12 illustrates directional displacement in the Y direction versus directional displacement in the X direction, wherein the predicted bead width was from models and taking the liquidus temperature to be around 1400 C.

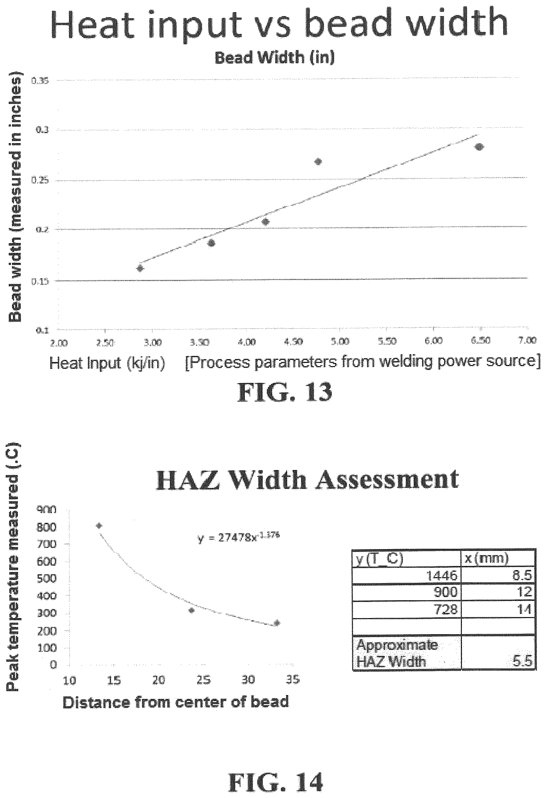

Turning to Fig. 13, a heat input versus bead width graph is illustrated. The heat input based on process parameters can be calculated. The bead width can be based on heat input from look-up tables. This can be converted into temperature contours such as shown in Fig. 12. Fig. 12 illustrates a heat contour that can be a prediction based on modeling and not by measurement. Fig. 12 illustrates how the thermal profile can look but often is inaccurately modeled because of the need to be measured in real time (which the subject application provides). Fig. 12 can be used to provide metrics such as heat affected zone width. For example, the heat affected zone width can be used to generate a tolerance, wherein the tolerance can be based on fitting the actual temperature measured to a distance from the center of the bead of the weld and suggest a different travel speed or wire feed speed in order to achieve the appropriate heat affected zone width.

Turning to Fig. 14, heat affected zone width information is illustrated. The heat input can be calculated based on process parameters. The bead width can be determined based on heat input from a look-up table or pre-defined by a user or stored on a memory. This information can be converted into a temperature contour as shown in Fig. 12 and metrics such as heat affected zone width can be determined. The subject innovation can allow a determination of an accurate estimate of heat affected zone (HAZ) width from actual temperature measurements and fitting the actual temperature measurements to a distance from the center of the bead. The "tolerance" could be based on this fit to the predicted heat countour and suggest a different travel speed or wire feed speed in order to achieve the appropriate HAZ width.

It is to be appreciated that each type of welding operation and/or each type of material of workpiece W can include a temperature contour, wherein the temperature contour can include temperatures for distances from arc 112 and/or locations on or within workpiece W. For each location or distance, a tolerance can be defined. The definition of each temperature contour can be at least one of pre-defined, dynamically created based on user input or computer monitoring, downloaded or communicated from a cloud computing service, pre-defined and later updated based on welding operations performed, or a combination thereof. The temperature contour can further include information such as, but not limited to, temperatures or tolerances based on at least one of a distance from arc 112 and/or locations on or within workpiece W, maximum temperature for a location on or within workpiece W, minimum temperature for a location on or within workpiece W, range of temperatures allowed for a location on or within workpiece W, temperature or tolerance for a location based on a welding operation, temperature or tolerance for a location based on a type of material of workpiece W, temperature or tolerance for a type of electrode, temperature or tolerance for a welding operation, among others.

Controller 102 can be configured to identify an average temperature of workpiece W based on receiving two or more temperature readings for one or more locations on or within workpiece W. Controller 102 can further communicate an indication when the average temperature of workpiece W exceeds a tolerance for the average temperature of such welding operation, and in particular, such workpiece. In a particular example, temperature sensor can detect a first temperature on a first location on the first side and detect a second temperature on a second location on the second side, wherein the first location on the first side is mirrored to the second location on the second side. In such particular example, controller can evaluate the average temperature of both the first temperature and the second temperature to a tolerance for the first location and/or the second location.

In another particular example, temperature sensor can detect a first temperature on a first location on the first side and detect a second temperature on a second location on the second side, wherein the first location on the first side is not mirrored to the second location on the second side. In such particular example, controller can evaluate the first temperature and/or the second temperature to a tolerance respective to each first location and/or the second location.

In still another embodiment, temperature sensor 108 can capture a baseline temperature of workpiece W to compare such baseline temperature to a temperature captured during the welding operation or after the welding operation. Such comparison can be used in evaluating the weld created and/or workpiece W and whether the weld created and/or workpiece W are suitable for a particular application or industry. In particular, controller 102 can be configured to evaluate a number of exceeded tolerances during a welding operation and generate a score which can be used to evaluate whether the weld created or workpiece W of the welding operation is suitable.

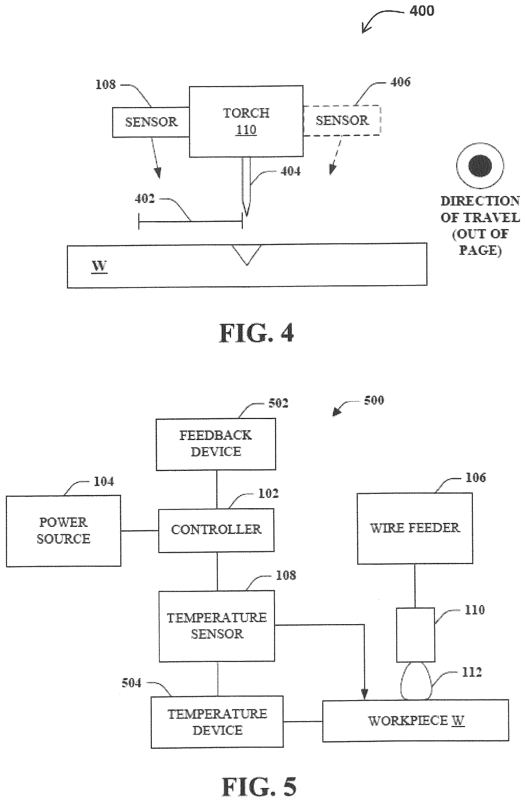

Fig. 4 illustrates an exemplary, non-limiting embodiment of welding torch 110 that includes temperature sensor 108 to detect temperature at a location that is a distance from an arc created between an electrode and workpiece W. Torch 110 is illustrated in a travel direction coming out of the page of Fig. 4. Temperature sensor 108 (referred to in Fig. 4 as "sensor 108") can be removeably attached or incorporated into torch 110. In a particular embodiment, sensor 108 can be affixed to a first side of torch 110 corresponding to a side of workpiece W. Sensor 108 can detect temperature at a location that is distance 402 away from at least one of electrode 404 or welding torch 110 (also referred to as "torch 110").

In another embodiment, sensor 108 can be removeably attached or incorporated into torch 110. In this particular embodiment, sensor 108 can be affixed to a first side of torch 110 corresponding to a side of workpiece W and additional sensor 406 can be affixed to a second side of torch 110 corresponding to a second side of workpiece W, wherein the first side of torch 110 is opposite the second side of torch 110 and the first side is opposite the second side. Sensor 108 and additional sensor 406 can detect temperatures at two or more locations that each have a respective distance away from at least one of electrode 404 or welding torch 110.

Fig. 5 illustrates an exemplary, non-limiting embodiment of welding system 500 that communicates an indication to feedback device 502 based on a measured temperature of workpiece W at a distance adjacent to a travel path of welding torch 110. System 500 includes welding torch 110 having an electrode in which power source 104 creates arc 112 between electrode and workpiece W to complete an electrical circuit to perform the welding operation. System 500 can include power source 104 that is configured to create arc 112 between an electrode and workpiece W and further include wire feeder 106 is configured to deliver welding wire to a puddle formed by the electrode. Controller 102 can be configured to manage wire feed speed (WFS) of wire feeder 106, power source 104 that creates arc 112 for the welding operation. It is to be appreciated that the system 500 can be used to perform a welding operation with a consumable electrode, a non-consumable electrode, a shielding gas, no shielding gas, or a combination thereof. Controller 102 is further configured to communicate an indication when a temperature detected by temperature sensor 108 exceeds or does not meet a tolerance. The indication can be communicated to an operator performing the welding operation in which the indication provides instruction to adjust wire feed speed and/or travel speed. In another embodiment, controller 102 adjusts the wire feed speed in addition to an operator being notified to adjust travel speed in response to the temperature exceeding or not meeting a tolerance. In still another embodiment, controller can adjust the travel speed and/or the wire feed speed in response to the detected temperature not meeting or exceeding a tolerance.

Controller 102 can communicate the indication to feedback device 502. Feedback device 502 can deliver the indication, wherein the indication can be, but is not limited to being auditory, visual, adaptive, haptic, among others. Feedback device 502 can be, but is not limited to, a speaker, a computer, a display, a cell phone, a tablet, a computing device, a siren, a light, an LED, a helmet, gloves, an electronic device, a wearable electronic device, or an apron. In a particular embodiment of the subject innovation, the indication can be a green light for within tolerance and a red light for outside tolerance, and such light can be flashed in a peripheral vision of the user via the helmet or welding lens of a helmet. In another embodiment, the feedback device 502 can be a heads-up-display (HUD) that provides an image and/or a sound to indicate the operator performing the welding operation should adjust at least one of a welding parameter, a wire feed speed, and/or a travel speed. In a particular example, a vibration can be used as an indication to an operator, wherein the vibration is provided by feedback device 502 being, such as, a welding torch, a glove, a wearable device, a welding helmet, a floor mat, a belt, an apron, among others. In still another example, the indication can be a light or LED, wherein feedback device 502 receives the indication from controller 102 and the light or LED can signal when a tolerance is met or exceeded. For example, colors for the light or LED can be designated to a particular indication (e.g., red for exceeding a tolerance, green for being within a tolerance). In another example, a HUD in a helmet can provide a graphic overlay with a heat map or infrared map of heat on the workpiece to illustrate heat input and/or tolerances being exceeded or not.

System 500 further includes temperature device 504 that is configured to deliver additional heat or cooling to workpiece W based at least on detected temperature at a location on or within workpiece W. The temperature device 504 can be a heating element to increase heat input to a portion of workpiece W or at an area of workpiece W or a cooling element to decrease heat input to a portion of workpiece W or at an area of workpiece W. For instance, temperature device 504 can be used to adjust heat input to workpiece W instead of adjusting wire feed speed or travel speed. In another instance, temperature device 504 can be used to adjust heat input to workpiece W in combination with adjusting wire feed speed and/or travel speed.

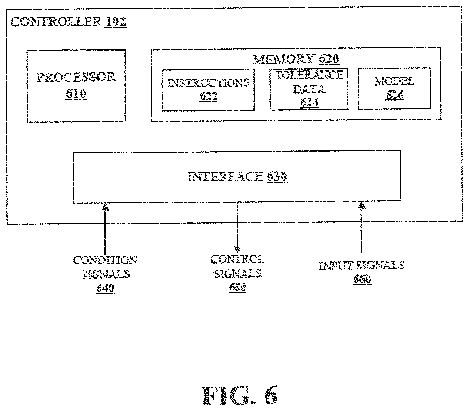

Referring to Fig. 6, illustrated a schematic block diagram of an exemplary, non-limiting embodiment of controller 102 according to one or more aspects. As shown in Fig. 6, controller 102 can be a microcontroller that includes a processor 610, a memory 620, and an interface 630. Processor 610 is configured to execute computer-executable instructions, such as instructions 622 stored by memory 620, for example. Instructions 622 comprise software executable by processor 610 to configure controller 102 to perform aspects described herein. Memory 620 can be non-transitory, computer-readable storage media including volatile storage media (e.g. a random access memory, a data cache, a register) and/or non-volatile storage media such as a hard drive, flash memory, portable media (e.g. floppy disk, USB drive, optical disc, etc.), a read-only memory, etc. For the purposes of this description, the various forms of computer-readable storage media described above are collectively shown and referred to as memory 620. Interface 630 can be a communications interface to enable controller 102 to communicate with other components such as welding power source 104, temperature sensor 108, etc. In one example, interface 630 can include general purpose input/output (l/O) pins, which can be coupled to various signal lines or circuit paths to transmit or receive signals. In another example, the interface 630 can be a connection to a data bus. In yet another example, interface 630 can be a wireless interface.

Controller 102, via interface 630, can receive condition signals 640 indicative of one or more conditions (e.g. environmental conditions, physical conditions, operational conditions, etc.) associated with the systems 100, 200, 400, and/or 500. Tolerance data 624, stored by memory 620, can be generated based on condition signals 640. Processor 610 can employ model 626 with tolerance data 624 to determine, for example, communications for a feedback based on a temperature of a workpiece, travel speed for a welding operation for a temperature detected by a temperature sensor, a wire feed speed for a welding operation for a temperature detected by a temperature sensor, among others. Based on these determined quantities or values, controller 102 can generate control signals 650 transmitted by interface 630. Control signals 650 can be transmitted to power source 104 to limit a welding output generated thereby, or to a user interface to inform an operator of input limits (e.g. welding output preset limits) and/or to normalize inputs in accordance with the limits. For instance, such limits can be, but are not limited to, a travel speed, a wire feed speed, a welding parameter, among others. Moreover, interface 630 can receive input signals 660 (temperature readings, travel speed readings, wire feed speed, for example), which can be utilized to generate or supplement tolerance data 624, or establish settings (e.g. output presets) by which controller 102 implements via control signal 650.

Model 626 can be a set of mathematical relationships correlating various conditions to temperature of the workpiece at a location that is a distance from the arc, temperature of a workpiece being a particular type of material, a distance from the arc for a workpiece, etc. as described above. Accordingly, processor 610 can utilize the set of mathematical relationships with tolerance data 624 to calculate deliverable power, travel speed, wire feed speed, or a welding parameter. In another example, model 626 can be based on empirical data. For instance, for the respective conditions and, specifically respective levels or values for the conditions, results can be experimentally measured and collected. The results can be, for example, actual measurements of temperatures under varying conditions for various types of materials that can be a workpiece. The results can be tabulated and the tables, which can be form of model 626, utilized to determine or interpolate desired quantities based on tolerance data 624 gathered by controller 102. In another example, the empirical data is utilized to generate to train model 626 via artificial intelligence or machine learning techniques. For instance, model 626 can be a neural network or other classification scheme that is trained on the empirical data to develop relationships between temperature of the workpiece and travel speed and/or wire feed speed. The developed relationships can be utilized to determine welding parameters from new condition inputs in situ. According to this example, model 626 can include or involve, for instance, a neural network, a decision tree, an association rule, a support vector machine, a Bayesian network, genetic algorithms, or the like.

By way of example and not limitation, the welding parameter can be, but is not limited to, a type of welding operation, a type of shielding gas, a material composition of workpiece W, a welding pattern, a type of electrode, a composition of electrode, a wire feed speed, a waveform used for the welding operation, a polarity of a welding wire, a type of flux, a number of electrodes used in the welding operation, an arc voltage, a travel speed of a tractor welder that performs the welding operation, a travel speed of a torch that performs the welding operation, an arc current level, a height of torch, a distance between workpiece W and torch or an end of the electrode, an oscillation width of electrode, a temperature of welding wire, a temperature of electrode, a type of material of workpiece W, a frequency of oscillation of electrode, a polarity of the arc current, a polarity of the current for welding wire, a parameter that affects an arc current of the welding operation, a gauge of wire, a material of wire, an oscillation dwell, a left oscillation dwell, a right oscillation dwell, one or more temperatures of workpiece W at one or more locations on workpiece W, any and all variation of advanced process controls (e.g., move controls, pulse-frequency, ramp rates, background level ratios, etc.), and the like.

In an embodiment, the indication informs an operator to adjust a travel speed of the welding torch along the weld joint to bring the temperature of the workpiece into compliance. In an embodiment, the system can include a wire feeder configured to deliver a welding wire to the arc, wherein the controller is configured to adjust a wire feed speed of the wire feeder when the temperature of the workpiece exceeds the tolerance. In an embodiment, the controller is configured to calibrate the tolerance based at least one of the material of the workpiece, a distance from the arc, or a welding process.

In an embodiment, the system can include the temperature sensor is further configured to detect an additional temperature of the workpiece at an additional path and the controller is further configured to communicate the indication to a feedback device when an average temperature of the temperature and the additional temperature exceed the tolerance.

In an embodiment, the path is a location that is aligned with the welding torch and parallel to a travel path of the welding torch. In an embodiment, the path is a location that is in front of the welding torch, a distance from the electrode, and parallel to a travel path of the welding torch. In an embodiment, the path is a location that is behind the welding torch, a distance from the electrode, and parallel to a travel path of the welding torch.

In an embodiment, the system can include the temperature sensor is affixed to the welding torch and detects the temperature of the workpiece along the path adjacent to the weld joint during the welding operation with the path being on a first side of the workpiece and a distance from the arc. In the embodiment, the system can include an additional temperature sensor affixed to the welding torch configured to detect an additional temperature of the workpiece along an additional path adjacent to the weld joint during the welding operation with the additional path being on a second side of the workpiece and the distance from the arc, the first side is opposite the second side with a travel path of the welding torch therebetween.

In the embodiment, the controller further configured to communicate the indication to the feedback device when an average temperature of the workpiece exceeds the tolerance associated with the material of the workpiece, wherein the average temperature is of the temperature on the path on the first side and the additional temperature on the additional path on the second side.

In an embodiment, the indication is a visual indicator and the feedback device is a helmet. In an embodiment, the indication is a haptic feedback and the feedback device is at least one of a glove or a welding torch.

In an embodiment, the controller increases the wire feed speed of the wire feeder when the temperature of the workpiece is below a minimum temperature used to calculate the tolerance. In an embodiment, the controller decreases the wire feed speed of the wire feeder when the temperature of the workpiece is above a maximum temperature used to calculate the tolerance.

In an embodiment, the system can further include the temperature sensor detects the temperature of the workpiece along the path adjacent to the weld joint during the welding operation with the path being a first distance from the arc; the temperature sensor detects an additional temperature of the workpiece along an additional path adjacent to the weld joint during the welding operation with the additional path being on a second distance from the arc; and the controller further configured to communicate the indication to the feedback device when the temperature or the additional temperature of the workpiece exceeds the tolerance associated with the material of the workpiece for the first distance or the second distance.

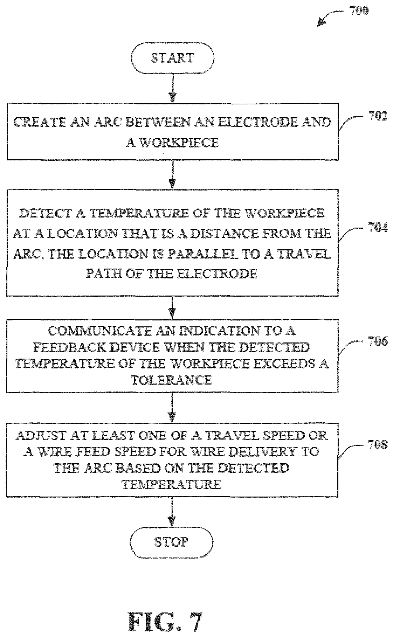

In view of the exemplary devices and elements described supra, methodologies that may be implemented in accordance with the disclosed subject matter will be better appreciated with reference to the flow charts and/or methodology of Figs. 7-8. The methodologies and/or flow diagrams are shown and described as a series of blocks, the claimed subject matter is not limited by the order of the blocks, as some blocks may occur in different orders and/or concurrently with other blocks from what is depicted and described herein. Moreover, not all illustrated blocks may be required to implement the methods and/or flow diagrams described hereinafter.

Sequentially, the following occurs as illustrated in the decision tree flow diagram 700 of Fig. 7 which is a flow diagram 700 that detects temperature of a workpiece to reduce inconsistency of heat input for a welding operation. At reference block 702, an arc between an electrode and a workpiece can be created. At reference block 704, a temperature of the workpiece at a location that is a distance from the arc can be detected, wherein the location is parallel to a travel path of the electrode. In particular, the temperature is continuously being detected in real time along a path that can be adjacent to the travel path of the electrode. In a particular example, the temperature can be detected along a path (or paths for more than one temperature sensor) that is parallel to the travel path of the electrode on either side of the electrode. At reference block 706, an indication can be communicated to a feedback device when the detected temperature of the workpiece exceeds a tolerance. At reference block 708, at least one of a travel speed or a wire feed speed for wire delivery to the arc can be adjusted based on the detected temperature. In particular, at least one of the wire feed speed or the travel speed can be maintained, increased, or decreased based on the detected temperature being within the tolerance, above a maximum temperature used to calculate the tolerance, or below a minimum temperature used to calculate the tolerance.

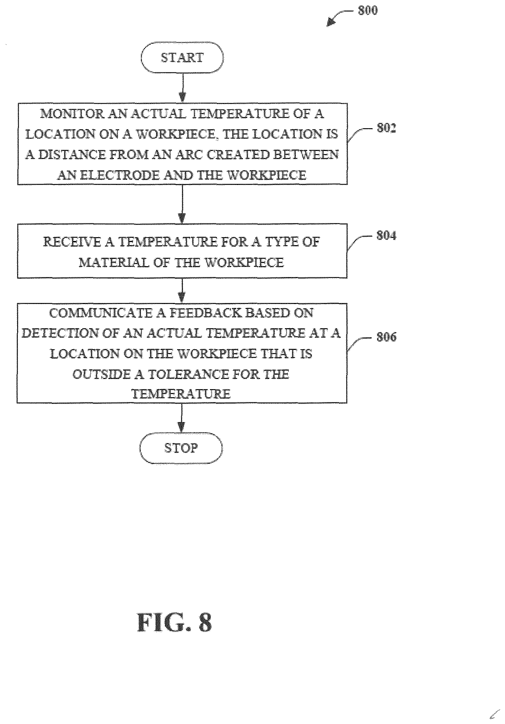

Fig. 8 illustrates a flow diagram 800 that communicates feedback based on a monitored temperature of a workpiece at a location that is a distance from the arc created on such workpiece. At reference block 802, an actual temperature of a location on a workpiece can be monitored, wherein the location is a distance from an arc created between an electrode and the workpiece. At reference block 804, a temperature for a type of material of the workpiece can be received. At reference block 806, a feedback can be communicated based on detection of an actual temperature at a location on the workpiece that is outside a tolerance for the temperature. For example, a tolerance can be based on a distance from an arc for a particular type of material of a workpiece and such tolerance can be a range of temperatures (e.g., a minimum temperature to a high temperature). In another example, the tolerance can be a percentage of a target temperature for a particular type of material of a workpiece at a specific distance (e.g., a ±five percent of a target temperature).

In an embodiment, the indication informs an operator to adjust a travel speed of a welding torch to bring the temperature of the workpiece into compliance of the tolerance.

In an embodiment, the method can include detecting an additional temperature of the workpiece at an additional location that is an additional distance from the arc, aligned with the arc, and parallel to the travel path of the electrode and communicating the indication to the feedback device when the detected temperature or additional temperature of the workpiece exceeds the tolerance for the distance or the additional distance.

In an embodiment, the method can include calibrating the tolerance based at least one of the type of material of the workpiece, a distance from the arc, or a welding process.

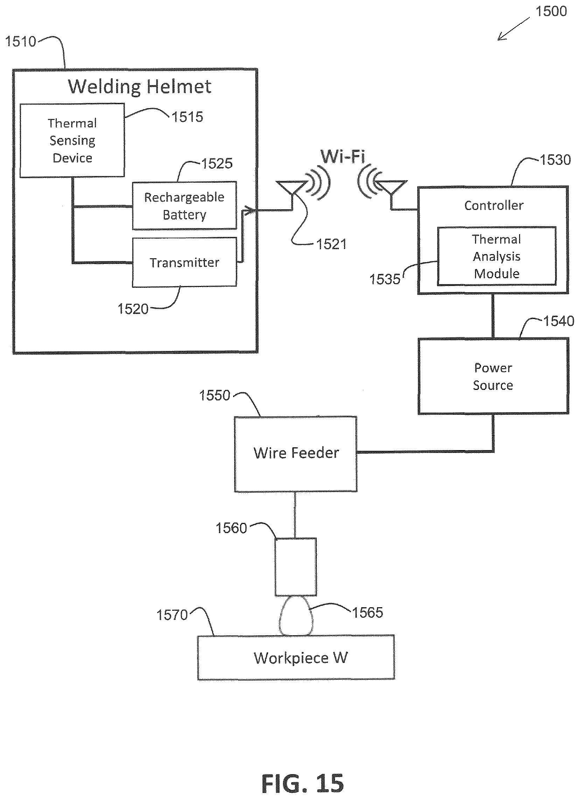

FIG. 15 illustrates a welding system 1500 for controlling welding parameters based on thermal characteristics of a weld environment. The weld environment may include, for example, a workpiece, an electrode, and a weld puddle. The welding system 1500 includes a welding helmet 1510 having a thermal sensing device 1515, a transmitter device 1520, and a rechargeable battery 1525. The rechargeable battery 1525 powers (i.e., provides electrical energy to) the thermal sensing device 1515 and the transmitter device 1520. The welding system 1500 also includes a controller 1530 having a thermal analysis module 1535. The welding system 1500 further includes a power source 1540, a wire feeder 1550, and a welding torch or gun 1560. During a welding operation, an electrode (e.g., a welding wire electrode) is provided to the torch 1560 by the wire feeder 1550 to create an arc 1565 between a tip of the electrode and a workpiece 1570. In one embodiment, the wire feeder 1550 is operatively connected to the power source 1540 and is configured to deliver a welding wire electrode toward the workpiece 1570 to the arc 1565 at a selected or controlled wire feed speed.

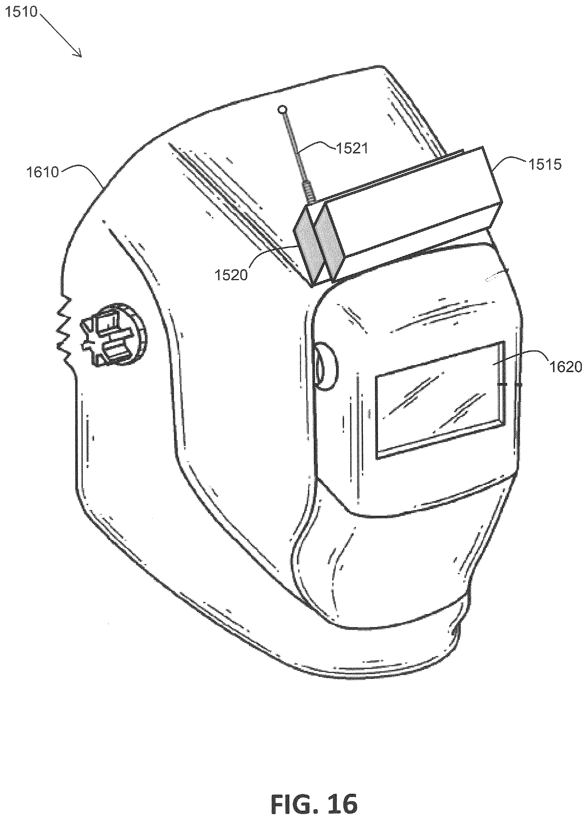

FIG. 16 illustrates the welding helmet 1510 that is a part of the welding system 1500 of FIG. 15. The welding helmet 1510 includes a shell 1610 having a filtered viewing window 1620. The welding helmet 1510 is configured to be worn by a human welder to protect the human welder as he/she views a weld environment through the viewing window 1620 during a welding operation. The filtered viewing window 1620 is filtered by an auto-darkening filter (ADF), in accordance with one configuration. Other types of filters are possible as well, in accordance with other configurations.

FIG. 16 shows the thermal sensing device 1515 and the transmitting device 1520 positioned at the top front portion of the welding helmet 1510 above the viewing window 1620. In general, the thermal sensing device 1515 and the transmitting device 1520 may be integrated with the shell 1610 in any of various ways, in accordance with various embodiments. For example, as shown in FIG. 16, the thermal sensing device 1515 is attached to the transmitting device 1520, and the transmitting device 1520 is attached to the shell 1610. An antenna 1521 is operatively attached to the transmitting device 1520 to facilitate radio frequency transmission.

The thermal sensing device 1515 is positioned to be able to "see" the weld environment during a welding operation. The weld environment includes at least a portion of the workpiece 1570, the electrode coming out of the torch 1560, and the weld puddle (not shown) formed by the arc 1565 on the workpiece during a welding operation. In one configuration, the thermal sensing device 1515 is a thermal imaging device that is capable of sensing infrared wavelengths of thermal energy. For example, the thermal sensing device 1515 may include an infrared temperature device, a thermography device, a thermal camera, a thermocouple device, a thermistor device, a resistance temperature detector (RTD), among others, in accordance with various alternative configurations. The thermal sensing device 1515 is configured to sense thermal energy of the weld environment and generate thermal data based on the sensed thermal energy during a welding operation. In this example, the thermal data may be in the form of, for example, temperatures, spatial thermal gradients, or temporal thermal gradients. Other forms of thermal data may be possible as well, in accordance with other embodiments.

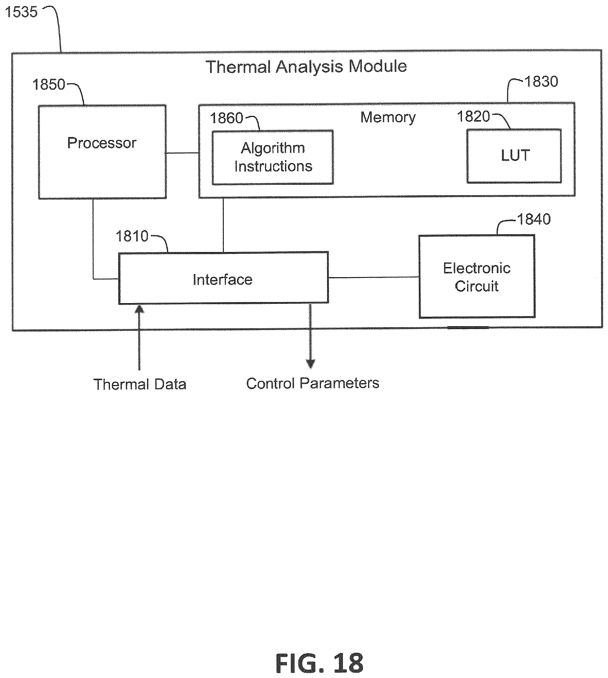

In one configuration, the transmitter device 1520 is a radio frequency transmitter that uses, for example, WiFi technology. Other wireless radio frequency technologies are possible as well (e.g., Bluetooth^{®}). The transmitter device 1520 is configured to wirelessly transmit the thermal data generated by the thermal sensing device 1515 to the controller 1530. The controller 1530 is configured to wirelessly receive the thermal data from the transmitter device, for example, via WiFi technology. The controller includes the thermal analysis module 1535 which is configured to analyze the thermal data to generate control parameters. The thermal analysis module 1535 is discussed in more detail later herein with respect to at least FIG. 18.

The control parameters include one or more of a current adjustment command, a voltage adjustment command, a waveform adjustment command, or a wire feed speed adjustment command. The control parameters (adjustment commands) may be sent from the controller 1530 to the power source 1540 to adjust or modify one or more welding parameters of the welding operation by a certain amount or percent from the present settings of the welding parameters. The welding parameters that are modified may include, for example, a welding current, a welding voltage, a welding waveform, or a wire feed speed. Other welding parameters are possible as well, in accordance with other embodiments. The control parameters may take other forms as well, in accordance with other embodiments. For example, the control parameters may include selectable set points of an absolute current, an absolute voltage, an absolute waveform, and an absolute wire feed speed. Furthermore, the welding waveform welding parameter may actually include a plurality of welding waveform parameters such as, for example, a waveform shape, a waveform pulse frequency, a waveform polarity, a waveform DC offset, a waveform balance, a waveform ramp rate, a waveform background level, etc., any of which may be adjusted by a corresponding control parameter from the controller 1530.

The thermal analysis module 1535 is configured to analyze the thermal data of the weld environment such that characteristics of the weld environment are controlled in a desired manner during a welding operation. For example, in one embodiment, the thermal analysis module 1535 analyzes the thermal data and generates control parameters which results in controlling an amount of heat input to a weld, compensating for the base material cooling too quickly or too slowly, changing the size of the heat-affected zone (HAZ), or some combination thereof. Other characteristics of the weld environment may be controlled as well, in accordance with other embodiments. For example, a temperature of the welding electrode may be controlled or a temperature of the weld puddle may be controlled, in accordance with other embodiments. Control of such characteristics of the weld environment is accomplished automatically and in real time. The human welder does not have to make decisions on how to change the weld parameters as he welds and does not have to stop and make such changes.

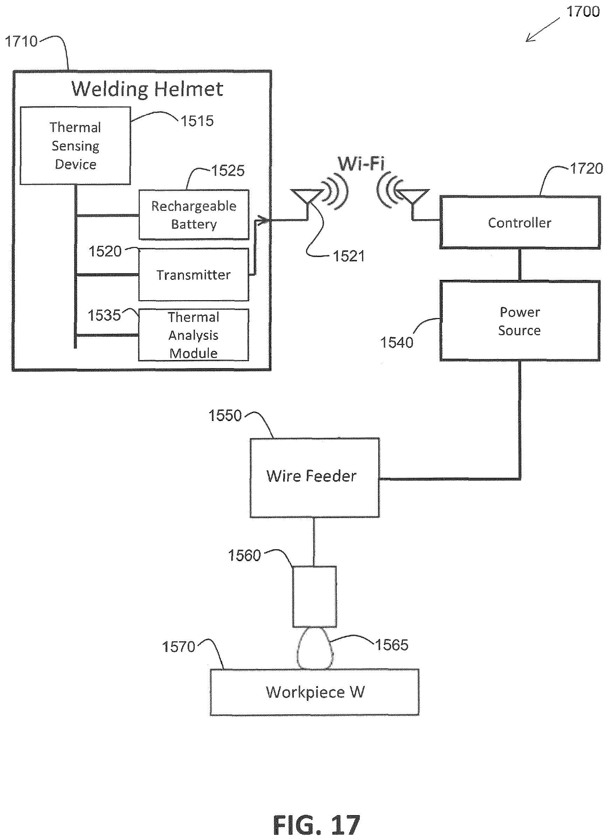

FIG. 17 illustrates an embodiment, according to the present invention, of a welding system 1700 for controlling welding parameters based on thermal characteristics of a weld environment. The welding system 1700 is similar to the welding system 1500 of FIG. 15, except that the thermal analysis module 1535 is integrated with the welding helmet 1710 instead of the controller 1720. The thermal sensing device 1515 provides the thermal data to the thermal analysis module 1535 and the thermal analysis module 1535 generates the control parameters based on an analysis of the thermal data. Instead of wirelessly transmitting the thermal data, the transmitter device 1520 transmits control commands to the controller 1720. The welding helmet 1710 is a "smart" welding helmet in the sense that the analysis of the thermal data is performed in the welding helmet 1710. In this manner, the controller 1720 is less complicated than the controller 1530 of FIG. 15. However, the controller 1720 is still able to wirelessly receive information (i.e., the control commands) from the welding helmet 1710.

Referring to FIG. 16, the welding helmet 1710 of FIG. 17 may be configured by integrating the thermal analysis module 1535 with the thermal sensing device 1515 which is attached to the transmitter device 1520, in accordance with one embodiment. Alternatively, the thermal analysis module 1535 may be integrated with the transmitter device 1520 which is attached to the shell 1610, in accordance with another embodiment. Other embodiments of integrating the thermal analysis module 1535 with the welding helmet are possible as well.

FIG. 18 illustrates possible configurations of a thermal analysis module 1535 which is a part of the welding system 1500 of FIG. 15 or the welding system 1700 (according to the present invention) of FIG. 17. For purposes of discussion with respect to FIG. 18, the term "thermal data" may refer to the raw thermal data from the thermal sensing device 1515 or data that is derived from the raw thermal data in some manner (e.g. pre-processed thermal data). The thermal analysis module 1535 includes an interface 1810 and a look-up table (LUT) 1820 stored in a memory 1830. The interface 1810 is configured to receive the thermal data and provide the thermal data as an addressable input to the memory 1830. The LUT 1820 stored in the memory 1830 is configured such that each addressable input provides one or more control parameters as an output of the memory 1830. The control parameters are output from the thermal analysis module 1535 via the interface 1810.

In accordance with another configuration, the thermal analysis module 1535 includes the interface 1810 and an electronic circuit 1840 (e.g., a logic circuit or a digital signal processor). The interface 1810 is configured to receive the thermal data and provide the thermal data as an input to the electronic circuit 1840. The electronic circuit 1840 is configured such that each input results in one or more control parameters being output from the electronic circuit 1840. The control parameters are then output from the thermal analysis module 1535 via the interface 1810.

In accordance with yet another configuration, the thermal analysis module 1535 includes the interface 1810, a processor 1850 and an algorithm 1860 implemented as a set of computer-executable instructions that are stored in the memory 1830 and configured to execute on the processor 1850. The interface 1810 is configured to receive the thermal data and provide the thermal data as an input to the processor 1850. The computer-executable instructions of the algorithm 1860 are loaded into the processor 1850 and the processor 1850 executes the algorithm to operate on the input (thermal data) to generate one or more control parameters as an output of the processor 1850. The control parameters are then output from the thermal analysis module 1535 via the interface 1810.

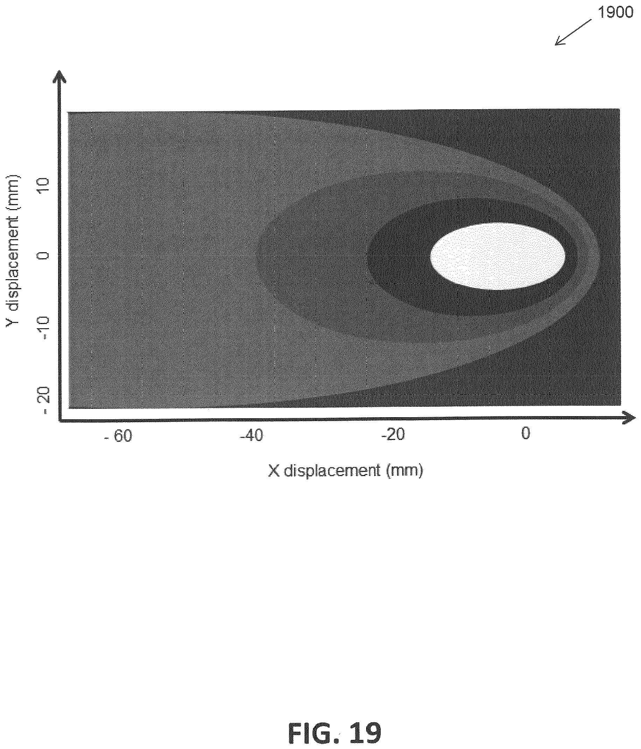

Whether implemented with a LUT, an electronic circuit, an algorithm, or some combination thereof, the thermal analysis module 1535 is configured to effectively analyze the input thermal data and generate output control parameters. For example, FIG. 19 illustrates a graphical representation of an example embodiment of pre-processed thermal data 1900 generated by the thermal sensing device 1515 of the welding system 1500 of FIG. 15, or the welding system 1700 (according to the present invention) of FIG. 17, and pre-processed by the thermal analysis module 1535. The pre-processed thermal data 1900 shows various temperature regions (as indicated by the different shaded regions) over a portion of the surface of the workpiece 1570. The various temperature regions were generated by the thermal analysis module 1535 by applying pre-defined temperature threshold ranges to temperature values of the pixels of the raw thermal temperature data out of the thermal sensing device 1515. Six (6) temperature regions are shown in FIG. 19 as six different shaded regions.

In accordance with one configuration, after the thermal analysis module 1535 pre-processes the raw thermal data from the thermal sensing device 1515, the thermal analysis module 1535 generates ratios of the sizes (e.g., number of pixels) of the various shaded regions representing the temperature regions. The resultant combination of ratios correlate to percentages that various welding parameters (e.g., welding current, welding voltage, welding waveform, and/or wire feed speed) are to be adjusted to maintain a desired temperature profile across the workpiece 1570. Such correlations may be determined via prior experimentation, for example. Subsequently, the thermal analysis module 1535 generates control parameters that will command the controller (1530 or 1720) to make the adjustments in the power source 1540 and/or the wire feeder 1550. In this manner, a desired temperature profile can be maintained across the workpiece 1570 during a welding operation.

Embodiments for controlling heat input for a weld or metal deposition process by monitoring thermal characteristics of a weld environment are disclosed. One embodiment includes a welding helmet 1510 having a shell 1610 to be worn by a human welder to protect the human welder while viewing a weld environment through a viewing window 1620 of the shell 1610 during a welding operation using a welding system 100. A thermal sensing device 1515 is integrated with the shell 1610 to sense thermal energy of the weld environment and generate thermal data based on the thermal energy. A thermal analysis module 1535 is integrated with the shell 1610 to analyze the thermal data and generate control parameters. A transmitter device 1520 is integrated with the shell 1610 to transmit the control parameters to the welding system 100. The control parameters control at least one welding parameter of the welding system 100 during the welding operation.

While the embodiments discussed herein have been related to the systems and methods discussed above, these embodiments are intended to be exemplary and are not intended to limit the applicability of these embodiments to only those discussions set forth herein. The control systems and methodologies discussed herein are equally applicable to, and can be utilized in, systems and methods related to arc welding, laser welding, brazing, soldering, plasma cutting, waterjet cutting, laser cutting, and any other systems or methods using similar control methodology, without departing from the spirit or scope of the above discussed inventions. The embodiments and discussions herein can be readily incorporated into any of these systems and methodologies by those of skill in the art. By way of example and not limitation, a power supply as used herein (e.g., welding power supply, among others) can be a power supply for a device that performs welding, arc welding, laser welding, brazing, soldering, plasma cutting, waterjet cutting, laser cutting, among others. Thus, one of sound engineering and judgment can choose power supplies other than a welding power supply departing from the intended scope of coverage of the embodiments of the subject invention.

The best mode for carrying out the invention has been described for purposes of illustrating the best mode known to the applicant at the time. The examples are illustrative only and not meant to limit the invention, as defined by the scope of the appended claims.

**REFERENCE NUMERALS**

| | | | |
|---|---|---|---|
| | | 708 | step |
| 100 | welding system | 800 | flow diagram |
| 102 | controller | 802 | step |
| 104 | power source | 804 | step |
| 106 | wire feeder | 806 | step |
| 108 | temperature sensor | 902 | temperature sensor (Al1) |
| 110 | welding torch | 904 | temperature sensor (AI2) |
| 112 | arc | 906 | temperature sensor (AI3) |
| 200 | welding system | 1500 | welding system |
| 202 | weld | 1510 | weldinq helmet |
| 204 | travel path | 1515 | thermal sensing device |
| 206 | reference numeral | 1520 | transmitter device |
| 208 | location | 1521 | antenna |
| 210 | location | 1525 | recharqeable battery |
| 214 | location | 1530 | controller |
| 402 | distance | 1535 | analysis module |
| 404 | electrode | 1540 | power source |
| 406 | sensor | 1550 | wire feeder |
| 500 | welding system | 1560 | welding qun |
| 502 | feedback device | 1565 | arc |
| 504 | temperature device | 1570 | workpiece |
| 610 | processor | 1610 | shell |
| 620 | memory | 1620 | viewinq window |
| 622 | instructions | 1700 | welding system |
| 624 | tolerance data | 1710 | welding helmet |
| 626 | model | 1720 | controller |
| 630 | interface | 1810 | interface |
| 640 | condition siqnals | 1820 | look-up table (LUT) |
| 650 | control siqnals | 1830 | memory |
| 660 | input siqnal | 1840 | electronic circuit |
| 700 | flow diagram | 1850 | processor |
| 702 | step | 1860 | algorithm |
| 704 | step | 1900 | data |
| 706 | step | W | workpiece |

## Claims

1. A welding helmet (1510), comprising:
a shell (1610), having a filtered viewing window (1620), configured to be worn by a human welder to protect the human welder as the human welder views a weld environment through the viewing window (1620) during a welding operation performed by the human welder using a welding system;
a thermal sensing device (1515) integrated with the shell (1610); and
a transmitter device (1520) integrated with the shell (1620);
**characterized by**
the thermal sensing device (1515) being configured to sense thermal energy of the weld environment and generate thermal data based on the thermal energy during the welding operation, wherein the weld environment includes at least one of a workpiece (W), an electrode, and a weld puddle, wherein the thermal data includes spatial thermal gradients and temporal thermal gradients; and
further **characterized by** a thermal analysis module (1535) integrated with the shell (1610), operatively connected to the thermal sensing device (1515), and configured to analyze the thermal data to generate control parameters; and
further **characterized in that**
the transmitter device (1520) is operatively connected to the thermal analysis module (1535), and configured to transmit the control parameters to the welding system (100), wherein the control parameters control at least one welding parameter of the welding system (100) during the welding operation.

2. The welding helmet of claim 1, wherein the thermal data includes temperatures of the weld environment; and/or wherein the control parameters include at least one of a current adjustment command, a voltage adjustment command, a waveform adjustment command, or a wire feed speed adjustment command; wherein the at least one welding parameter includes a welding current, a welding voltage, a welding waveform, or a wire feed speed.

3. The welding helmet of claim 1 or 2, further comprising a rechargeable battery (1525) integrated with the shell (1610) and configured to provide electrical energy to the thermal sensing device (1515), the thermal analysis module (1535), and/or the transmitter device (1520); and/or wherein the thermal analysis module (1535) is configured as a look-up table (1820) stored in a memory (1830), using the thermal data (1900) as an addressable input to the memory (1830) and providing the control parameters as an output of the memory (1830).

4. The welding helmet of one of the claims 1 to 3, wherein the thermal analysis module (1535) comprises: a processor (1850); a memory (1830); and an algorithm (1860) implemented as a set of computer-executable instructions stored in the memory (1830) and configured to execute on the processor (1850); and/or
wherein the thermal analysis module (1535) comprises an electronic circuit (1840) configured to use the thermal data (1900) as an input to the electronic circuit (1840) and provide the control parameters as an output of the electronic circuit (1840).

5. The welding helmet of one of the claims 1 to 4, further comprising an antenna (1521) operatively connected to the transmitter device (1520), wherein the transmitter device (1520) is configured to wirelessly transmit the control parameters to a controller (102) or a power source (104) of the welding system (100) via the antenna (1521); and/or further comprising a communication cable configured to be connected between the transmitter device (1520) and a controller (102) or a power source (104) of the welding system (100), wherein the transmitter device is configured to transmit the control parameters to the controller (102) or the power source (104) of the welding system (100) via the communication cable.

6. A welding system (100), comprising:
a power source (104) configured to output a welding current to an electrode to create an arc (112) between the electrode and a workpiece (W) to form a weld puddle during a welding operation; and a
welding helmet (1510) according to one of the preceding claims.

7. The welding system of claim 6, wherein the thermal data includes at least one of temperatures, spatial thermal gradients, or temporal thermal gradients of the weld environment.

8. The welding system of claim 6 or 7, wherein the control parameters include at least one of a current adjustment command, a voltage adjustment command, a waveform adjustment command, or a wire feed speed adjustment command.

9. The welding system of one of the claims 6 to 8, wherein the at least one welding parameter includes the welding current, a welding voltage, a welding waveform, or a wire feed speed.

10. The welding system of one of the claims 6 to 9, further comprising a rechargeable battery (1525) integrated with the welding helmet (1510) and configured to provide electrical energy to the thermal sensing device (1515) and the transmitter device (1520).

11. The welding system of one of the claims 6 to 10, further comprising a wire feeder (106) operatively connected to the power source (104) and configured to deliver a welding wire toward the workpiece (W) to the arc at a wire feed speed, wherein the welding wire is the electrode.

12. The welding system of one of the claims 6 to 11, wherein the thermal analysis module (1535) is configured as a look-up table (1820) stored in a memory (1830) of the controller (1720), using the thermal data (1900) as an addressable input to the memory (1830) and providing the control parameters as an output of the memory (1830).

13. The welding system of one of the claims 6 to 12, wherein the thermal analysis module (1535) comprises an algorithm (1860) implemented as a set of computer-executable instructions stored in a memory (1830) of the controller (1720) and configured to execute on a processor (1850) of the controller (1720).

14. The welding system of one of the claims 6 to 13, wherein the thermal analysis module (1535) comprises an electronic circuit (1840) configured to use the thermal data (1900) as an input to the electronic circuit (1840) and provide the control parameters as an output of the electronic circuit (1840).

15. The welding system of one of the claims 6 to 14, further comprising a welding torch (110) configured to accommodate the electrode and be held by the human welder to direct the electrode to the workpiece (W).

## Patentansprüche

1. Schweißerhelm (1510), der umfasst:
eine Schale (1610), die ein mit einem Filter ausgestattetes Sichtfenster (1620) aufweist und dafür konfiguriert ist, von einem menschlichen Schweißer getragen zu werden, um den menschlichen Schweißer zu schützen, wenn der menschliche Schweißer eine Schweißumgebung durch das Sichtfenster (1620) während eines Schweißvorgangs betrachtet, der durch den menschlichen Schweißer unter Verwendung eines Schweißsystems durchgeführt wird;
eine Wärmefühlvorrichtung (1515), die in die Schale (1610) integriert ist; und
eine Sendervorrichtung (1520), die in die Schale (1620) integriert ist;
**dadurch gekennzeichnet, dass**
die Wärmefühlvorrichtung (1515) dafür konfiguriert ist, Wärmeenergie der Schweißumgebung abzufühlen und Wärmedaten auf der Grundlage der Wärmeenergie während des Schweißvorgangs zu generieren, wobei die Schweißumgebung mindestens eines von einem Werkstück (W), einer Elektrode und einer Schweißpfütze umfasst, wobei die Wärmedaten räumliche Wärmegradienten und zeitliche Wärmegradienten enthalten; und
des Weiteren **gekennzeichnet durch**
ein Wärmeanalysemodul (1535), das in die Schale (1610) integriert ist, mit der Wärmefühlvorrichtung (1515) wirkverbunden ist, und dafür konfiguriert ist, die Wärmedaten zu analysieren, um Steuerungsparameter zu generieren; und
des Weiteren **dadurch gekennzeichnet, dass**
die Sendervorrichtung (1520) mit dem Wärmeanalysemodul (1535) wirkverbunden ist und dafür konfiguriert ist, die Steuerungsparameter an das Schweißsystem (100) zu übertragen, wobei die Steuerungsparameter mindestens einen Schweißparameter des Schweißsystems (100) während des Schweißvorgangs steuern.

2. Schweißhelm nach Anspruch 1, wobei die Wärmedaten Temperaturen der Schweißumgebung enthalten; und/oder
wobei die Steuerungsparameter mindestens eines von einem Stromjustierbefehl, einem Spannungsjustierbefehl, einem Wellenformjustierbefehl und einem Drahtvorschubgeschwindigkeitsjustierbefehl enthalten;
wobei der mindestens eine Schweißparameter einen Schweißstrom, eine Schweißspannung, eine Schweißwellenform oder eine Drahtvorschubgeschwindigkeit enthält.

3. Schweißhelm nach Anspruch 1 oder 2, der des Weiteren eine wiederaufladbare Batterie (1525) umfasst, die in die Schale (1610) integriert und dafür konfiguriert ist, die Wärmefühlvorrichtung (1515), das Wärmeanalysemodul (1535) und/oder die Sendervorrichtung (1520) mit elektrischer Energie zu versorgen; und/oder
wobei das Wärmeanalysemodul (1535) als eine in einem Speicher (1830) gespeicherte Nachschlagetabelle (1820) konfiguriert ist, die die Wärmedaten (1900) als eine adressierbare Eingabe in den Speicher (1830) verwendet und die Steuerungsparameter als eine Ausgabe des Speichers (1830) bereitstellt.

4. Schweißhelm nach einem der Ansprüche 1 bis 3, wobei das Wärmeanalysemodul (1535) umfasst:
einen Prozessor (1850);
einen Speicher (1830); und
einen Algorithmus (1860), der als ein Satz computerausführbarer Instruktionen implementiert ist, die in dem Speicher (1830) gespeichert und dafür konfiguriert sind, in dem Prozessor (1850) ausgeführt zu werden; und/oder
wobei das Wärmeanalysemodul (1535) eine elektronische Schaltung (1840) umfasst, die dafür konfiguriert ist, die Wärmedaten (1900) als eine Eingabe in die elektronische Schaltung (1840) zu verwenden und die Steuerungsparameter als eine Ausgabe der elektronischen Schaltung (1840) bereitzustellen.

5. Schweißhelm nach einem der Ansprüche 1 bis 4, der des Weiteren eine Antenne (1521) umfasst, die mit der Sendervorrichtung (1520) wirkverbunden ist, wobei die Sendervorrichtung (1520) dafür konfiguriert ist, die Steuerungsparameter über die Antenne (1521) drahtlos an einen Controller (102) oder eine Stromquelle (104) des Schweißsystems (100) zu übertragen; und/oder
des Weiteren ein Kommunikationskabel umfasst, das dafür konfiguriert ist, zwischen der Sendervorrichtung (1520) und einem Controller (102) oder einer Stromquelle (104) des Schweißsystems (100) verbunden zu werden, wobei die Sendervorrichtung dafür konfiguriert ist, die Steuerungsparameter über das Kommunikationskabel an den Controller (102) oder die Stromquelle (104) des Schweißsystems (100) zu übertragen.

6. Schweißsystem (100), das umfasst:
eine Stromquelle (104), die dafür konfiguriert ist, einen Schweißstrom an eine Elektrode auszugeben, um einen Lichtbogen (112) zwischen der Elektrode und einem Werkstück (W) zu erzeugen, um während eines Schweißvorgangs ein Schweißpfütze zu bilden; und
einen Schweißhelm (1510) nach einem der vorangehenden Ansprüche.

7. Schweißsystem nach Anspruch 6, wobei die Wärmedaten mindestens eines von Temperaturen, räumlichen Wärmegradienten und zeitlichen Wärmegradienten der Schweißumgebung enthalten.

8. Schweißsystem nach Anspruch 6 oder 7, wobei die Steuerungsparameter mindestens eines von einem Stromjustierbefehl, einem Spannungsjustierbefehl, einem Wellenformjustierbefehl und einem Drahtvorschubgeschwindigkeitsjustierbefehl enthalten.

9. Schweißsystem nach einem der Ansprüche 6 bis 8, wobei der mindestens eine Schweißparameter einen Schweißstrom, eine Schweißspannung, eine Schweißwellenform oder eine Drahtvorschubgeschwindigkeit enthält.

10. Schweißsystem nach einem der Ansprüche 6 bis 9, das des Weiteren eine wiederaufladbare Batterie (1525) umfasst, die in den Schweißhelm (1510) integriert und dafür konfiguriert ist, die Wärmefühlvorrichtung (1515) und die Sendervorrichtung (1520) mit elektrischer Energie zu versorgen.

11. Schweißsystem nach einem der Ansprüche 6 bis 10, das des Weiteren eine Drahtzufuhrvorrichtung (106) umfasst, die mit der Stromquelle (104) wirkverbunden und dafür konfiguriert ist, einen Schweißdraht mit einer Drahtvorschubgeschwindigkeit in Richtung des Werkstücks (W) zu dem Lichtbogen zuzuführen, wobei der Schweißdraht die Elektrode ist.

12. Schweißsystem nach einem der Ansprüche 6 bis 11, wobei das Wärmeanalysemodul (1535) als eine Nachschlagetabelle (1820) konfiguriert ist, die in einem Speicher (1830) des Controllers (1720) gespeichert ist und die Wärmedaten (1900) als eine adressierbare Eingabe in den Speicher (1830) verwendet und die Steuerungsparameter als eine Ausgabe des Speichers (1830) bereitstellt.

13. Schweißsystem nach einem der Ansprüche 6 bis 12, wobei das Wärmeanalysemodul (1535) einen Algorithmus (1860) umfasst, der als ein Satz computerausführbarer Instruktionen implementiert ist, die in einem Speicher (1830) des Controllers (1720) gespeichert und dafür konfiguriert sind, in einem Prozessor (1850) des Controllers (1720) ausgeführt zu werden.

14. Schweißsystem nach einem der Ansprüche 6 bis 13, wobei das Wärmeanalysemodul (1535) eine elektronische Schaltung (1840) umfasst, die dafür konfiguriert ist, die Wärmedaten (1900) als eine Eingabe in die elektronische Schaltung (1840) zu verwenden und die Steuerungsparameter als eine Ausgabe der elektronischen Schaltung (1840) bereitzustellen.

15. Schweißsystem nach einem der Ansprüche 6 bis 14, das des Weiteren einen Schweißbrenner (110) umfasst, der dafür konfiguriert ist, die Elektrode aufzunehmen und durch den menschlichen Schweißer gehalten zu werden, um die Elektrode auf das Werkstück (W) zu richten.

## Revendications

1. Casque de soudage (1510), comprenant :
une coque (1610), ayant une fenêtre de visualisation filtrée (1620), configurée pour être portée par un soudeur humain pour protéger le soudeur humain lorsque le soudeur humain visualise un environnement de soudage à travers la fenêtre de visualisation (1620) au cours d'une opération de soudage réalisée par le soudeur humain en utilisant un système de soudage ;
un dispositif de détection thermique (1515) intégré à la coque (1610) ; et
un dispositif émetteur (1520) intégré à la coque (1620) ;
**caractérisé par**
le dispositif de détection thermique (1515) étant configuré pour détecter une énergie thermique de l'environnement de soudage et générer des données thermiques sur la base de l'énergie thermique au cours de l'opération de soudage, dans lequel l'environnement de soudage inclut au moins l'un parmi une pièce d'ouvrage (W), une électrode et un bain de fusion, dans lequel les données thermiques incluent des gradients thermiques spatiaux et des gradients thermiques temporels ; et
**caractérisé en outre par**
un module d'analyse thermique (1535) intégré à la coque (1610), relié opérationnellement au dispositif de détection thermique (1515), et configuré pour analyser les données thermiques pour générer des paramètres de commande ;
et **caractérisé en outre en ce que**
le dispositif émetteur (1520) est relié opérationnellement au module d'analyse thermique (1535), et configuré pour émettre les paramètres de commande au système de soudage (100), dans lequel les paramètres de commande commandent au moins un paramètre de soudage du système de soudage (100) au cours de l'opération de soudage.

2. Casque de soudage selon la revendication 1, dans lequel les données thermiques incluent des températures de l'environnement de soudage ; et/ou dans lequel les paramètres de commande incluent au moins l'une parmi une commande d'ajustement de courant, une commande d'ajustement de tension, une commande d'ajustement de forme d'onde, ou une commande d'ajustement de vitesse d'avance de fil ; dans lequel l'au moins un paramètre de soudage inclut un courant de soudage, une tension de soudage, une forme d'onde de soudage ou une vitesse d'avance de fil.

3. Casque de soudage selon la revendication 1 ou 2, comprenant en outre une batterie rechargeable (1525) intégrée à la coque (1610) et configurée pour fournir de l'énergie électrique au dispositif de détection thermique (1515), au module d'analyse thermique (1535) et/ou au dispositif émetteur (1520) ; et/ou dans lequel le module d'analyse thermique (1535) est configuré sous la forme d'une table de consultation (1820) stockée dans une mémoire (1830), utilisant les données thermiques (1900) en tant qu'une entrée adressable dans la mémoire (1830) et fournissant les paramètres de commande en tant qu'une sortie de la mémoire (1830).

4. Casque de soudage selon l'une des revendications 1 à 3, dans lequel le module d'analyse thermique (1535) comprend : un processeur (1850) ; une mémoire (1830) ; et un algorithme (1860) mis en œuvre en tant qu'un jeu d'instructions exécutables par ordinateur stockées dans la mémoire (1830) et configurées pour s'exécuter sur le processeur (1850) ; et/ou
dans lequel le module d'analyse thermique (1535) comprend un circuit électronique (1840) configuré pour utiliser les données thermiques (1900) en tant qu'une entrée dans le circuit électronique (1840) et fournir les paramètres de commande en tant qu'une sortie du circuit électronique (1840).

5. Casque de soudage selon l'une des revendications 1 à 4, comprenant en outre une antenne (1521) reliée opérationnellement au dispositif émetteur (1520), dans lequel le dispositif émetteur (1520) est configuré pour émettre sans fil les paramètres de commande à un dispositif de commande (102) ou à une source d'énergie (104) du système de soudage (100) via l'antenne (1521) ; et/ou comprenant en outre un câble de communication configuré pour être relié entre le dispositif émetteur (1520) et un dispositif de commande (102) ou une source d'énergie (104) du système de soudage (100), dans lequel le dispositif émetteur est configuré pour émettre les paramètres de commande au dispositif de commande (102) ou à la source d'énergie (104) du système de soudage (100) via le câble de communication.

6. Système de soudage (100), comprenant :
une source d'énergie (104) configurée pour délivrer un courant de soudage à une électrode pour créer un arc (112) entre l'électrode et une pièce d'ouvrage (W) pour former un bain de fusion au cours d'une opération de soudage ; et
un casque de soudage (1510) selon l'une des revendications précédentes.

7. Système de soudage selon la revendication 6, dans lequel les données thermiques incluent au moins l'un parmi des températures, des gradients thermiques spatiaux ou des gradients thermiques temporels de l'environnement de soudage.

8. Système de soudage selon la revendication 6 ou 7, dans lequel les paramètres de commande incluent au moins l'une parmi une commande d'ajustement de courant, une commande d'ajustement de tension, une commande d'ajustement de forme d'onde, ou une commande d'ajustement de vitesse d'avance de fil.

9. Système de soudage selon l'une des revendications 6 à 8, dans lequel l'au moins un paramètre de soudage inclut le courant de soudage, une tension de soudage, une forme d'onde de soudage ou une vitesse d'avance de fil.

10. Système de soudage selon l'une des revendications 6 à 9, comprenant en outre une batterie rechargeable (1525) intégrée au casque de soudage (1510) et configurée pour fournir de l'énergie électrique au dispositif de détection thermique (1515) et au dispositif émetteur (1520) .

11. Système de soudage selon l'une des revendications 6 à 10, comprenant en outre une tête de soudage (106) reliée opérationnellement à la source d'énergie (104) et configurée pour amener un fil de soudage vers la pièce d'ouvrage (W) jusqu'à l'arc à une vitesse d'avance de fil, dans lequel le fil de soudage est l'électrode.

12. Système de soudage selon l'une des revendications 6 à 11, dans lequel le module d'analyse thermique (1535) est configuré sous la forme d'une table de consultation (1820) stockée dans une mémoire (1830) du dispositif de commande (1720), utilisant les données thermiques (1900) en tant qu'une entrée adressable dans la mémoire (1830) et fournissant les paramètres de commande en tant qu'une sortie de la mémoire (1830).

13. Système de soudage selon l'une des revendications 6 à 12, dans lequel le module d'analyse thermique (1535) comprend un algorithme (1860) mis en œuvre en tant qu'un jeu d'instructions exécutables par ordinateur stockées dans une mémoire (1830) du dispositif de commande (1720) et configurées pour s'exécuter sur un processeur (1850) du dispositif de commande (1720).

14. Système de soudage selon l'une des revendications 6 à 13, dans lequel le module d'analyse thermique (1535) comprend un circuit électronique (1840) configuré pour utiliser les données thermiques (1900) en tant qu'une entrée dans le circuit électronique (1840) et fournir les paramètres de commande en tant qu'une sortie du circuit électronique (1840).

15. Système de soudage selon l'une des revendications 6 à 14, comprenant en outre un chalumeau (110) configuré pour accueillir l'électrode et être tenu par le soudeur humain pour diriger l'électrode vers la pièce d'ouvrage (W) .
